# EUROPEAN PATENT APPLICATION

(11) **EP 0 905 518 A1**
(43) Date of publication of application: **31.03.1999**
(21) Application number: 97202909.4
(22) Date of filing: 23.09.1997
(51) Int. Cl.: G01N 33/68, G01N 33/50, G01N 33/564, C07K 7/08, C07K 7/06, C07K 16/16, C12N 15/05

(54) **Peptides specific for gluten-sensitive T-cells and use thereof**

(71) Applicant: Academisch Ziekenhuis Leiden, 2300 RC Leiden (NL); RIJKSUNIVERSITEIT LEIDEN, 2312 AV Leiden (NL)
(72) Inventor: Koning, Frits, 2353 WR Leiderdorp (NL); van de Wal, Yvonne, 2312 HX Leiden (NL); Drijfhout, Jan Wouter, 2321 AK Leiden (NL); Kooy-Winkelaar, Engelina Maria Christina, 2408 HL Alphen aan de Rijn (NL)
(74) Representative: Smulders, Theodorus A.H.J., Ir.

(57) **Abstract**

The invention relates to the field of immunology, more specifically to food-related immune enteropathies (gluten-sensitivities) such as celiac sprue, tropical sprue, giardiasis and food allergies of childhood, but also to disorders such as dermatitis herpetiformis (DH). The invention provides a method to find or characterise peptides that are recognised by intestinally derived gluten-specific T-cell which is instrumental in gluten sensitivity. The invention also provides such peptides which can be obtained from a prolamine, such as a gliadin, secalin, hordein, avenin and glutenins and provides peptides constituting a T-cell epitope obtainable from gliadin and glutenin, for example comprising the sequence SGQGSFQPSQQ or GQQGYYPTSPQQSGQ or derivatives thereof having similar properties.

(1) GENERAL INFORMATION:

(i) APPLICANT:
(A) NAME: Academisch Ziekenhuis Leiden
(B) STREET: Rijnsburgerweg 10
(C) CITY: Leiden
(D) STATE: Zuid-Holland
(E) COUNTRY: The Netherlands
(F) POSTAL CODE (ZIP): 2300 RC

(A) NAME: Rijksuniversiteit Leiden
(B) STREET: Stationsweg 46
(C) CITY: Leiden
(D) STATE: Zuid-Holland
(E) COUNTRY: The Netherlands
(F) POSTAL CODE (ZIP): 2312 AV

(ii) TITLE OF INVENTION: Peptides specific for gluten-sensitive
T-cells

(iii) NUMBER OF SEQUENCES: 129

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97202909.4

(2) INFORMATION FOR SEQ ID NO: 1:

(i) SEQUENCE CHARACTERISTICS:
(A) LENGTH: 4 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 1:

Pro Ser Gln Gln
1

(2) INFORMATION FOR SEQ ID NO: 2:

(i) SEQUENCE CHARACTERISTICS:
(A) LENGTH: 4 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 2:

Ser Pro Gln Gln
1

(2) INFORMATION FOR SEQ ID NO: 3:

(i) SEQUENCE CHARACTERISTICS:
(A) LENGTH: 4 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 3:

Pro Ser Asp Gln
1

(2) INFORMATION FOR SEQ ID NO: 4:

(i) SEQUENCE CHARACTERISTICS:
(A) LENGTH: 4 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 4:

Pro Ser Glu Gln
1

(2) INFORMATION FOR SEQ ID NO: 5:

(i) SEQUENCE CHARACTERISTICS:
(A) LENGTH: 4 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 5:

Pro Ser Ser Gln
1

(2) INFORMATION FOR SEQ ID NO: 6:

(i) SEQUENCE CHARACTERISTICS:
(A) LENGTH: 11 amino acids

(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 6:

Ser Gly Gln Gly Ser Phe Gln Pro Ser Gln Gln
1               5                   10

(2) INFORMATION FOR SEQ ID NO: 7:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 7:

Gly Gln Gln Gly Tyr Tyr Pro Thr Ser Pro Gln Gln Ser Gln Gln
1               5                   10                  15

(2) INFORMATION FOR SEQ ID NO: 8:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 8:

Gly Gln Gln Gly Tyr Tyr Pro Thr Ser Pro Gln Gln Ser Gly Gln
1               5                   10                  15

(2) INFORMATION FOR SEQ ID NO: 9:

(i) SEQUENCE CHARACTERISTICS:
(A) LENGTH: 25 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 9:

Gln Gln Pro Gln Gln Gln Tyr Pro Ser Gly Gln Gly Ser Phe Gln Pro
1               5                   10                  15

Ser Gln Gln Asn Pro Gln Ala Gln Gly
            20                  25

(2) INFORMATION FOR SEQ ID NO: 10:

(i) SEQUENCE CHARACTERISTICS:
(A) LENGTH: 18 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 10:

Gln Gln Pro Gln Gln Gln Tyr Pro Ser Gly Gln Gly Ser Phe Gln Pro
1               5                   10                  15

Ser Gln

(2) INFORMATION FOR SEQ ID NO: 11:

(i) SEQUENCE CHARACTERISTICS:
(A) LENGTH: 18 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 11:

Gln Gln Tyr Pro Ser Gly Gln Gly Ser Phe Gln Pro Ser Gln Gln Asn
1               5                   10                  15

Pro Gln

(2) INFORMATION FOR SEQ ID NO: 12:

(i) SEQUENCE CHARACTERISTICS:
(A) LENGTH: 18 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 12:

Pro Ser Gly Gln Gly Ser Phe Gln Pro Ser Gln Gln Asn Pro Gln Ala
1               5                   10                  15

Gln Gly

(2) INFORMATION FOR SEQ ID NO: 13:

(i) SEQUENCE CHARACTERISTICS:
(A) LENGTH: 10 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 13:

Pro Ser Gly Gln Gly Ser Phe Gln Pro Ser
1               5                   10

(2) INFORMATION FOR SEQ ID NO: 14:

(i) SEQUENCE CHARACTERISTICS:
(A) LENGTH: 11 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 14:

Pro Ser Gly Gln Gly Ser Phe Gln Pro Ser Gln
1               5                   10

(2) INFORMATION FOR SEQ ID NO: 15:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 15:

Pro Ser Gly Gln Gly Ser Phe Gln Pro Ser Gln Gln
1               5                   10

(2) INFORMATION FOR SEQ ID NO: 16:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 16:

Ser Gly Gln Gly Ser Phe Gln Pro Ser Gln Gln Asn
1               5                   10

(2) INFORMATION FOR SEQ ID NO: 17:

(i) SEQUENCE CHARACTERISTICS:
(A) LENGTH: 11 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 17:

Gly Gln Gly Ser Phe Gln Pro Ser Gln Gln Asn
1               5                   10

(2) INFORMATION FOR SEQ ID NO: 18:

(i) SEQUENCE CHARACTERISTICS:
(A) LENGTH: 10 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 18:

Gln Gly Ser Phe Gln Pro Ser Gln Gln Asn
1               5                   10

(2) INFORMATION FOR SEQ ID NO: 19:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 19:

Ser Gly Gln Gly Phe Phe Gln Pro Ser Gln Gln Asn
1               5                   10

(2) INFORMATION FOR SEQ ID NO: 20:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 20:

Ser Ser Gln Gly Ser Phe Gln Pro Ser Gln Gln Asn
1               5                   10

(2) INFORMATION FOR SEQ ID NO: 21:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 21:

Leu Gly Gln Gly Ser Phe Arg Pro Ser Gln Gln Asn
1               5                   10

(2) INFORMATION FOR SEQ ID NO: 22:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 22:

Ser Gly Gln Val Ser Phe Gln Ser Ser Gln Gln Asn
1               5                   10

(2) INFORMATION FOR SEQ ID NO: 23:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 23:

Ser Ser Gln Val Ser Phe Gln Pro Ser Gln Leu Asn
1               5                   10

(2) INFORMATION FOR SEQ ID NO: 24:

(i) SEQUENCE CHARACTERISTICS:
(A) LENGTH: 12 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 24:

Ser Val Gln Gly Val Tyr Gln Pro Gln Gln Gln Ser
1               5                   10

(2) INFORMATION FOR SEQ ID NO: 25:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 25:

Gly Gln Pro Gly Tyr Tyr Pro Thr Ser Pro Gln Gln Ser Gly Gln
1               5                   10                  15

(2) INFORMATION FOR SEQ ID NO: 26:

(i) SEQUENCE CHARACTERISTICS:
(A) LENGTH: 15 amino acids
(B) TYPE: amino acid
(C) STRANDEDNESS: unknown

(D) TOPOLOGY: unknown

(ii) MOLECULE TYPE: peptide

(iii) HYPOTHETICAL: NO

(xi) SEQUENCE DESCRIPTION: SEQ ID NO: 26:

Gly Gln Leu Gly Tyr Tyr Pro Thr Ser Pro Gln Gln Ser Gly Gln
1               5                   10                  15

(2) INFORMATION FOR SEQ ID NO: 27:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 27:

Gly Gln Ser Gly Tyr Tyr Pro Thr Ser Pro Gln Gln Ser Gly Gln
1               5                   10                  15

(2) INFORMATION FOR SEQ ID NO: 28:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 28:

Gly Gln Gln Gly Tyr Tyr Pro Ile Ser Pro Gln Gln Ser Gly Gln
1               5                   10                  15

(2) INFORMATION FOR SEQ ID NO: 29:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 29:

Gly Gln Gln Gly Tyr Tyr Pro Thr Ser Ser Gln Gln Ser Gly Gln
1               5                   10                  15

(2) INFORMATION FOR SEQ ID NO: 30:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 30:

Gly Gln Gln Gly Tyr Tyr Pro Thr Ser Leu Gln Gln Ser Gly Gln
1               5                   10                  15

(2) INFORMATION FOR SEQ ID NO: 31:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 31:

Gly Gln Gln Gly Tyr Tyr Pro Thr Ser Pro Gln Gln Leu Gly Gln
1               5                   10                  15

(2) INFORMATION FOR SEQ ID NO: 32:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 32:

Gly Gln Gln Gly Tyr Tyr Pro Thr Ser Pro Gln Gln Pro Gly Gln
1               5                   10                  15

(2) INFORMATION FOR SEQ ID NO: 33:

(i) SEQUENCE CHARACTERISTICS:
(A) LENGTH: 15 amino acids

(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 33:

Gly Gln Gln Gly Tyr Tyr Pro Thr Ser Pro Gln Gln Ser Pro Gln
1               5                   10                  15

(2) INFORMATION FOR SEQ ID NO: 34:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 34:

Gly Gln Gln Gly Tyr Tyr Pro Thr Ser Val Gln Gln Pro Gly Gln
1               5                   10                  15

(2) INFORMATION FOR SEQ ID NO: 35:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 35:

Gly Gln Ala Gly Tyr Tyr Pro Thr Ser Pro Gln Gln Ser Gly Gln
1               5                   10                  15

(2) INFORMATION FOR SEQ ID NO: 36:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 36:

Gly Gln Gln Gly Tyr Tyr Pro Ile Ser Ala Gln Gln Leu Gly Gln
1               5                   10                  15

(2) INFORMATION FOR SEQ ID NO: 37:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 37:

Gly Gln Pro Arg Tyr Tyr Pro Thr Ser Pro Gln Gln Ser Gly Gln
1               5                   10                  15

(2) INFORMATION FOR SEQ ID NO: 38:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 38:

Gly Gln Pro Gly Tyr Tyr Pro Thr Ser Pro Gln Gln Pro Gly Gln
1               5                   10                  15

(2) INFORMATION FOR SEQ ID NO: 39:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 39:

Gly Gln Gln Gly Tyr Tyr Pro Thr Ser Pro Gln Gln Pro Pro Gln
1               5                   10                  15

(2) INFORMATION FOR SEQ ID NO: 40:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 40:

Gly Gln Pro Gly Tyr Tyr Leu Thr Ser Pro Gln Gln Ser Gly Gln
1               5                   10                  15

(2) INFORMATION FOR SEQ ID NO: 41:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 41:

Gly Gln Pro Gly Tyr Tyr Pro Thr Ser Pro Gln Gln Ile Gly Gln
1               5                   10                  15

(2) INFORMATION FOR SEQ ID NO: 42:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 42:

Gly Gln Pro Gly Tyr Tyr Pro Thr Ser Leu Gln Gln Ser Gly Gln
1               5                   10                  15

(2) INFORMATION FOR SEQ ID NO: 43:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 43:

Gly Gln Pro Gly Tyr Tyr Pro Thr Ser Pro Leu Gln Ser Gly Gln
1               5                   10                  15

(2) INFORMATION FOR SEQ ID NO: 44:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 44:

Gly Gln His Gly Tyr Tyr Pro Thr Ser Pro Gln Leu Ser Gly Gln
1               5                   10                  15

(2) INFORMATION FOR SEQ ID NO: 45:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 45:

Gly Gln Ser Gly Tyr Tyr Pro Thr Ser Pro Gln Gln Pro Gly Gln
1               5                   10                  15

(2) INFORMATION FOR SEQ ID NO: 46:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 46:

Gly Gln Gln Gly His Cys Pro Thr Ser Pro Gln Gln Ser Gly Gln
1               5                   10                  15

(2) INFORMATION FOR SEQ ID NO: 47:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 47:

Gly Gln Gln Glu Tyr Tyr Leu Thr Ser Pro Gln Gln Ser Gly Gln
1               5                   10                  15

(2) INFORMATION FOR SEQ ID NO: 48:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 48:

Gly Gln Gln Gly Tyr Tyr Pro Ile Ser Leu Gln Gln Ser Gly Gln
1               5                   10                  15

(2) INFORMATION FOR SEQ ID NO: 49:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 49:

Gly Gln Gln Gly Tyr Tyr Pro Thr Ser Leu His Gln Ser Gly Gln
1               5                   10                  15

(2) INFORMATION FOR SEQ ID NO: 50:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 50:

Gly Gln Gln Gly Tyr Tyr Pro Ile Ser Pro Gln Gln Pro Gly Gln
1               5                   10                  15

(2) INFORMATION FOR SEQ ID NO: 51:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 51:

Gly Gln Gln Gly Tyr Tyr Pro Ile Ser Pro Gln Gln Leu Gly Gln
1               5                   10                  15

(2) INFORMATION FOR SEQ ID NO: 52:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 52:

Gly Gln Gln Gly Tyr Tyr Pro Thr Ser Leu Gln Gln Thr Gly Gln
1               5                   10                  15

(2) INFORMATION FOR SEQ ID NO: 53:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 53:

Gly Gln Gln Gly Tyr Tyr Pro Thr Ser Leu Gln Gln Pro Gly Gln

1                    5                    10                    15

(2) INFORMATION FOR SEQ ID NO: 54:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 54:

Gly Gln Gln Gly Tyr Tyr Pro Thr Ser Gln Gln Gln Pro Gly Gln
1               5                   10                  15

(2) INFORMATION FOR SEQ ID NO: 55:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 55:

Gly Gln Gln Gly Tyr Tyr Pro Thr Ser Leu Gln Gln Pro Gly Gln
1               5                   10                  15

(2) INFORMATION FOR SEQ ID NO: 56:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 56:

Gly Gln Gln Gly Tyr Tyr Pro Thr Ser Pro Gln His Pro Gly Gln
1               5                   10                  15

(2) INFORMATION FOR SEQ ID NO: 57:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 57:

Gly Gln Gln Gly Tyr Tyr Pro Thr Ser Pro Gln His Thr Gly Gln
1               5                   10                  15

(2) INFORMATION FOR SEQ ID NO: 58:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 58:

Gly Lys Gln Gly Tyr Tyr Pro Thr Ser Leu Gln Gln Pro Gly Gln
1               5                   10                  15

(2) INFORMATION FOR SEQ ID NO: 59:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 59:

Gly Gln Ser Gly Tyr Phe Pro Thr Ser Arg Gln Gln Ser Gly Gln
1               5                   10                  15

(2) INFORMATION FOR SEQ ID NO: 60:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 60:

Gly Gln Ser Gly Tyr Tyr Pro Thr Ser Ala Gln Gln Pro Gly Gln
1               5                   10                  15

(2) INFORMATION FOR SEQ ID NO: 61:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 61:

Gly Gln Ser Gly Tyr Tyr Leu Thr Ser Pro Gln Gln Leu Gly Gln
1               5                   10                  15

(2) INFORMATION FOR SEQ ID NO: 62:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 62:

Gly Gln Pro Gly Tyr Tyr Pro Thr Ser Pro Leu Gln Pro Gly Gln
1               5                   10                  15

(2) INFORMATION FOR SEQ ID NO: 63:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 63:

Gly Gln Pro Arg Tyr Tyr Pro Thr Ser Pro Gln Gln Pro Gly Gln
1               5                   10                  15

(2) INFORMATION FOR SEQ ID NO: 64:

(i) SEQUENCE CHARACTERISTICS:
(A) LENGTH: 15 amino acids
(B) TYPE: amino acid

(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 64:

Gly Gln Pro Gly Tyr Tyr Ser Thr Ser Pro Gln Gln Leu Gly Gln
1               5                   10                  15

(2) INFORMATION FOR SEQ ID NO: 65:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 65:

Gly Gln Pro Gly Tyr Tyr Pro Thr Ser Leu Gln Gln Leu Gly Gln
1               5                   10                  15

(2) INFORMATION FOR SEQ ID NO: 66:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 66:

Gly Gln Pro Trp Tyr Tyr Pro Thr Ser Pro Gln Glu Ser Gly Gln
1               5                   10                  15

(2) INFORMATION FOR SEQ ID NO: 67:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 67:

Gly Gln Pro Gly Tyr Asp Pro Thr Ser Pro Gln Gln Pro Gly Gln
1               5                   10                  15

(2) INFORMATION FOR SEQ ID NO: 68:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 68:

Gly Gln His Gly His Cys Pro Thr Ser Pro Gln Gln Ser Gly Gln
1               5                   10                  15

(2) INFORMATION FOR SEQ ID NO: 69:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 69:

Gly Gln His Trp Tyr Tyr Pro Thr Ser Pro Gln Leu Ser Gly Gln
1               5                   10                  15

(2) INFORMATION FOR SEQ ID NO: 70:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 70:

Gly Gln Pro Gly Tyr Asp Pro Thr Ser Pro Gln Gln Pro Gly Gln
1               5                   10                  15

(2) INFORMATION FOR SEQ ID NO: 71:

(i) SEQUENCE CHARACTERISTICS:

(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 71:

Gly Gln Gln Trp Tyr Tyr Pro Thr Ser Leu Gln Gln Pro Gly Gln
1               5                   10                  15

(2) INFORMATION FOR SEQ ID NO: 72:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 72:

Gly Gln Gln Ala Tyr Tyr Pro Thr Ser Ser Gln Gln Ser Arg Gln
1               5                   10                  15

(2) INFORMATION FOR SEQ ID NO: 73:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 73:

Gly Gln Gln Gly His Tyr Pro Thr Ser Leu Gln Gln Leu Gly Gln
1               5                   10                  15

(2) INFORMATION FOR SEQ ID NO: 74:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 74:

Gly Gln Gln Gly His Tyr Pro Thr Ser Leu Gln Gln Pro Gly Gln
1               5                   10                  15

(2) INFORMATION FOR SEQ ID NO: 75:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 75:

Gly Gln Gln Gly His Cys Pro Thr Ser Pro Gln Gln Thr Gly Gln
1               5                   10                  15

(2) INFORMATION FOR SEQ ID NO: 76:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 76:

Gly Gln Gln Gly Tyr Tyr Pro Ser Ser Leu Gln Gln Pro Gly Gln
1               5                   10                  15

(2) INFORMATION FOR SEQ ID NO: 77:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 77:

Gly Gln Gln Gly Tyr Tyr Pro Thr Ser Leu Trp Gln Pro Gly Gln
1               5                   10                  15

(2) INFORMATION FOR SEQ ID NO: 78:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 78:

Gly Gln Gln Gly Tyr Tyr Pro Thr Ser Leu Gln His Thr Gly Gln
1               5                   10                  15

(2) INFORMATION FOR SEQ ID NO: 79:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 79:

Gly Gln Pro Gly Tyr Tyr Pro Thr Ser Ser Gln Gln Pro Thr Gln
1               5                   10                  15

(2) INFORMATION FOR SEQ ID NO: 80:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 80:

Gly Gln Pro Gly Tyr Tyr Pro Thr Ser Ser Gln Gln Pro Thr Gln
1               5                   10                  15

(2) INFORMATION FOR SEQ ID NO: 81:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 81:

Gly Gln Pro Gly Tyr Tyr Leu Thr Ser Pro Leu Gln Leu Gly Gln
1               5                   10                  15

(2) INFORMATION FOR SEQ ID NO: 82:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 82:

Gly Gln Pro Gly Tyr Tyr Ser Ser Ser Pro Gln Gln Leu Gly Gln
1               5                   10                  15

(2) INFORMATION FOR SEQ ID NO: 83:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 83:

Gly Gln Pro Gly Tyr Tyr Pro Thr Ser Leu Arg Gln Pro Gly Gln
1               5                   10                  15

(2) INFORMATION FOR SEQ ID NO: 84:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 84:

Gly Gln Pro Gly Tyr Tyr Pro Thr Ser Pro Trp Gln Pro Glu Gln
1               5                   10                  15

(2) INFORMATION FOR SEQ ID NO: 85:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 85:

Gly Gln Pro Gly His Tyr Pro Thr Ser Pro Leu Gln Pro Gly Gln
1               5                   10                  15

(2) INFORMATION FOR SEQ ID NO: 86:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 86:

Gly Gln Pro Arg Tyr Tyr Pro Thr Ser Ser Gln Gln Pro Gly Gln
1               5                   10                  15

(2) INFORMATION FOR SEQ ID NO: 87:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 87:

Gly Gln His Trp Tyr Tyr Pro Thr Ser Pro Lys Leu Ser Gly Gln
1               5                   10                  15

(2) INFORMATION FOR SEQ ID NO: 88:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 88:

Gly Gln Gln Gly His Cys Pro Met Ser Pro Gln Gln Thr Gly Gln
1               5                   10                  15

(2) INFORMATION FOR SEQ ID NO: 89:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 89:

Gly Gln Gln Gly His Tyr Pro Ala Ser Leu Gln Gln Val Gly Gln
1               5                   10                  15

(2) INFORMATION FOR SEQ ID NO: 90:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 90:

Gly Gln Gln Gly His Tyr Pro Ala Ser Gln Gln Gln Pro Gly Gln
1               5                   10                  15

(2) INFORMATION FOR SEQ ID NO: 91:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 91:

Gly Gln Gln Gly His Tyr Pro Ala Ser Leu Gln Gln Pro Gly Gln
1               5                   10                  15

(2) INFORMATION FOR SEQ ID NO: 92:

    (i) SEQUENCE CHARACTERISTICS:
        (A) LENGTH: 15 amino acids
        (B) TYPE: amino acid
        (C) STRANDEDNESS: unknown
        (D) TOPOLOGY: unknown

   (ii) MOLECULE TYPE: peptide

  (iii) HYPOTHETICAL: NO

   (xi) SEQUENCE DESCRIPTION: SEQ ID NO: 92:

Gly Gln Gln Gly His Tyr Pro Ala Ser Gln Gln Glu Pro Gly Gln
1               5                   10                  15

(2) INFORMATION FOR SEQ ID NO: 93:

    (i) SEQUENCE CHARACTERISTICS:
        (A) LENGTH: 15 amino acids
        (B) TYPE: amino acid
        (C) STRANDEDNESS: unknown
        (D) TOPOLOGY: unknown

   (ii) MOLECULE TYPE: peptide

  (iii) HYPOTHETICAL: NO

   (xi) SEQUENCE DESCRIPTION: SEQ ID NO: 93:

Gly Gln Gln Gly Gln Tyr Pro Ala Ser Gln Gln Gln Pro Gly Gln
1               5                   10                  15

(2) INFORMATION FOR SEQ ID NO: 94:

    (i) SEQUENCE CHARACTERISTICS:
        (A) LENGTH: 15 amino acids
        (B) TYPE: amino acid
        (C) STRANDEDNESS: unknown
        (D) TOPOLOGY: unknown

   (ii) MOLECULE TYPE: peptide

  (iii) HYPOTHETICAL: NO

   (xi) SEQUENCE DESCRIPTION: SEQ ID NO: 94:

Gly Gln Gln Gly Tyr Tyr Ser Ser Ser Leu Gln Gln Pro Gly Gln
1               5                   10                  15

(2) INFORMATION FOR SEQ ID NO: 95:

    (i) SEQUENCE CHARACTERISTICS:
        (A) LENGTH: 15 amino acids
        (B) TYPE: amino acid
        (C) STRANDEDNESS: unknown

(D) TOPOLOGY: unknown

(ii) MOLECULE TYPE: peptide

(iii) HYPOTHETICAL: NO

(xi) SEQUENCE DESCRIPTION: SEQ ID NO: 95:

Gly Gln Gln Gly Tyr Tyr Pro Ile Ser Ser Ala Gln Leu Gly Gln
1               5                   10                  15

(2) INFORMATION FOR SEQ ID NO: 96:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 96:

Gly Leu Gln Gly His Tyr Pro Ala Ser Leu Gln Gln Pro Gly Gln
1               5                   10                  15

(2) INFORMATION FOR SEQ ID NO: 97:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 97:

Gly Leu Pro Gly Tyr Tyr Pro Thr Ser Ser Leu Gln Pro Glu Gln
1               5                   10                  15

(2) INFORMATION FOR SEQ ID NO: 98:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 98:

Gly Gln Pro Gly Tyr Tyr Pro Thr Ser Ser Leu Gln Pro Glu Gln
1               5                   10                  15

(2) INFORMATION FOR SEQ ID NO: 99:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 99:

Gly Gln Gln Arg His Tyr Pro Ala Ser Leu Gln Gln Pro Gly Gln
1               5                   10                  15

(2) INFORMATION FOR SEQ ID NO: 100:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 100:

Gly Gln Gln Gly His Tyr Leu Ala Ser Gln Gln Gln Pro Gly Gln
1               5                   10                  15

(2) INFORMATION FOR SEQ ID NO: 101:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 101:

Gly Gln Gln Gly His Tyr Thr Ala Ser Leu Gln Gln Pro Gly Gln
1               5                   10                  15

(2) INFORMATION FOR SEQ ID NO: 102:

(i) SEQUENCE CHARACTERISTICS:
(A) LENGTH: 15 amino acids

(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 102:

Gly Gln Gln Gly His Tyr Leu Ala Ser Gln Gln Gln Pro Gly Gln
1               5                   10                  15

(2) INFORMATION FOR SEQ ID NO: 103:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 103:

Gly Gln Gln Gly His Tyr Leu Ala Ser Gln Gln Gln Pro Ala Gln
1               5                   10                  15

(2) INFORMATION FOR SEQ ID NO: 104:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 104:

Gly Gln Gln Gly Gln Ile Pro Ala Ser Gln Gln Gln Pro Gly Gln
1               5                   10                  15

(2) INFORMATION FOR SEQ ID NO: 105:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 105:

Gly Gln Gln Gly Gln Gln Pro Ala Gln Gly Gln Gln Gly Gln Gln
1               5                   10                  15

(2) INFORMATION FOR SEQ ID NO: 106:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 106:

Gly Gln Gln Gly Gln Gln Pro Gly Gln Gly Glu Gln Gly Gln Gln
1               5                   10                  15

(2) INFORMATION FOR SEQ ID NO: 107:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 107:

Gly Gln Gln Gly Gln Gln Pro Gly Gln Gly Gln Gln Ser Gly Gln
1               5                   10                  15

(2) INFORMATION FOR SEQ ID NO: 108:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 108:

Gly Gln Gln Gly Gln Gln Pro Gly Gln Gly Gln Arg Pro Gly Gln
1               5                   10                  15

(2) INFORMATION FOR SEQ ID NO: 109:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 109:

Gly Gln Gln Gly Gln Gln Pro Gly Gln Lys Gln Gln Ser Gly Gln
1               5                   10                  15

(2) INFORMATION FOR SEQ ID NO: 110:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 110:

Gly Gln Gln Gly Gln Gln Pro Gly Gln Gly Gln Gln Pro Gly Gln
1               5                   10                  15

(2) INFORMATION FOR SEQ ID NO: 111:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 111:

Gly Gln Gln Gly Gln Gln Pro Glu Gln Gly Gln Gln Gly Gln Gln
1               5                   10                  15

(2) INFORMATION FOR SEQ ID NO: 112:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 112:

Gly Gln Gln Gly Gln Gln Pro Glu Arg Gly Gln Gln Gly Gln Gln
1               5                   10                  15

(2) INFORMATION FOR SEQ ID NO: 113:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 113:

Gly Gln Gln Gly Gln Gln Ser Gly Gln Gly Gln Gln Leu Gly Gln
1               5                   10                  15

(2) INFORMATION FOR SEQ ID NO: 114:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 114:

Gly Gln Gln Gly Gln Gln Pro Gly Gln Gly Gln Gln Gly Gln Gln
1               5                   10                  15

(2) INFORMATION FOR SEQ ID NO: 115:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 115:

Gly Gln Gln Gly Gln Gln Pro Gly Glu Gly Gln Gln Gly Gln Gln
1               5                   10                  15

(2) INFORMATION FOR SEQ ID NO: 116:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 116:

Gly Glu Gln Gly Gln Gln Pro Gly Gln Gly Gln Gln Gly Gln Gln
1               5                   10                  15

(2) INFORMATION FOR SEQ ID NO: 117:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 117:

Gly Gln Lys Gly Gln Gln Pro Gly Gln Gly Gln Gln Pro Gly Gln
1               5                   10                  15

(2) INFORMATION FOR SEQ ID NO: 118:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 118:

Gly Gln Gln Gly Gln Gln Pro Ala Gln Val Gln Gln Gly Gln Gln
1               5                   10                  15

(2) INFORMATION FOR SEQ ID NO: 119:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 119:

Gly Gln Gln Ala Gln Gln Pro Gly Gln Gly Gln Gln Pro Gly Gln
1               5                   10                  15

(2) INFORMATION FOR SEQ ID NO: 120:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 120:

Gly Gln Gln Gly Gln Gln Pro Gly Gln Gly Gln Gln Pro Ala Gln
1               5                   10                  15

(2) INFORMATION FOR SEQ ID NO: 121:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 121:

Gly Gln Gln Gly Gln Gln Pro Gly Gln Gly Gln Gln Ser Gly Gln
1               5                   10                  15

(2) INFORMATION FOR SEQ ID NO: 122:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 122:

Gly Gln Gln Gly His Gln Pro Gly Gln Gly Gln Gln Ser Gly Gln

1                5                    10                   15

(2) INFORMATION FOR SEQ ID NO: 123:

    (i) SEQUENCE CHARACTERISTICS:
        (A) LENGTH: 15 amino acids
        (B) TYPE: amino acid
        (C) STRANDEDNESS: unknown
        (D) TOPOLOGY: unknown

    (ii) MOLECULE TYPE: peptide

    (iii) HYPOTHETICAL: NO

    (xi) SEQUENCE DESCRIPTION: SEQ ID NO: 123:

    Gly Gln Gln Thr Gly Gln Pro Gly Gln Lys Gln Gln Pro Gly Gln
    1               5                   10                  15

(2) INFORMATION FOR SEQ ID NO: 124:

    (i) SEQUENCE CHARACTERISTICS:
        (A) LENGTH: 15 amino acids
        (B) TYPE: amino acid
        (C) STRANDEDNESS: unknown
        (D) TOPOLOGY: unknown

    (ii) MOLECULE TYPE: peptide

    (iii) HYPOTHETICAL: NO

    (xi) SEQUENCE DESCRIPTION: SEQ ID NO: 124:

    Gly Gln Gln Gly Gln Gln Pro Arg Gln Trp Gln Gln Ser Gly Gln
    1               5                   10                  15

(2) INFORMATION FOR SEQ ID NO: 125:

    (i) SEQUENCE CHARACTERISTICS:
        (A) LENGTH: 15 amino acids
        (B) TYPE: amino acid
        (C) STRANDEDNESS: unknown
        (D) TOPOLOGY: unknown

    (ii) MOLECULE TYPE: peptide

    (iii) HYPOTHETICAL: NO

    (xi) SEQUENCE DESCRIPTION: SEQ ID NO: 125:

    Gly Gln Gln Gly His Gln Pro Gly Gln Gly Gln Gln Ser Gly Gln
    1               5                   10                  15

(2) INFORMATION FOR SEQ ID NO: 126:

    (i) SEQUENCE CHARACTERISTICS:
        (A) LENGTH: 15 amino acids
        (B) TYPE: amino acid
        (C) STRANDEDNESS: unknown
        (D) TOPOLOGY: unknown

(ii) MOLECULE TYPE: peptide

(iii) HYPOTHETICAL: NO

(xi) SEQUENCE DESCRIPTION: SEQ ID NO: 126:

Gly Gln Gln Gly Gln Gln Pro Gly Gln Gly Gln Gln Pro Gly Gln
1               5                   10                  15

(2) INFORMATION FOR SEQ ID NO: 127:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 127:

Gly Gln Gln Gly Gln Gln Pro Gly Ser Ala Gln Gln Gly Gln Gln
1               5                   10                  15

(2) INFORMATION FOR SEQ ID NO: 128:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 128:

Gly Gln Gln Ala Gln Gln Pro Gly Gln Gly Gln Gln Pro Gly Gln
1               5                   10                  15

(2) INFORMATION FOR SEQ ID NO: 129:

(i) SEQUENCE CHARACTERISTICS:
(A) LENGTH: 15 amino acids
(B) TYPE: amino acid
(C) STRANDEDNESS: unknown
(D) TOPOLOGY: unknown

(ii) MOLECULE TYPE: peptide

(iii) HYPOTHETICAL: NO

(xi) SEQUENCE DESCRIPTION: SEQ ID NO: 129:

Gly Gln Gln Gly Gln Gln Pro Gly Gln Ala Gln Gln Gly Gln Gln
1               5                   10                  15

## Description

The invention relates to the field of immunology, more specifically to food-related immune enteropathies such as celiac sprue, tropical sprue, giardiasis and food allergies of childhood, but also to disorders such as dermatitis herpetiformis (DH).

Celiac disease (CD) or celiac sprue is a disorder of the small intestine characterised by crypt-cell hyperplasia and villous atrophy, accompanied by an increased number of intraepithelial lymphocytes. Characteristic symptoms are a mild to severe malabsorption syndrome, diarrhoea, cachexia and weight loss but can sometimes include lymphoma or cancer. The disease is caused by a sensitivity to gluten (prolamine) and is precipitated in susceptible individuals by ingestion of cereal proteins. Cereal, in this application, relates to grain or related grasses or plants that produce it and to the (prepared) foodstuff. In particular wheat gluten, but also rye, and to a lesser extent barley and oat may cause disease. Current therapy of CD mainly involves dietary treatment of gluten-sensitive patients with diets lacking cereal compounds such as flour, which deprives these patients of such typical staple foods as for example bread. Wheat gluten comprises a mixture of two proteins, glutenins and gliadins, which contain 35-45% glutamine (Q) and 12-20% proline (P). Glutenins are of high molecular weight, comprising from 500-1000 amino acid molecules, covalently bound head-to-tail by disulfide bridges, forming multimeric complexes. Glutenins are responsible for the elasticity and extensibility of the gluten. The gliadines are of lower molecular weight, comprising from 250 to 600 amino acids, are monomeric, and are responsible for the viscosity of the gluten.

The gluten sensitivity is in general directed at the low molecular weight gliadins in wheat and analogous proteins in other edible grasses, for example secalins (rye), hordeins (barley) and avenins (oats); and more specifically at peptide fragments of these proteins. It is generally thought that especially N-terminal peptides of these proteins contain repetitive sequences (rich in P and Q residues) that are highly immunogenic and are disease precipitating epitopes. Major disease precipitating gliadins are known to contain repetitive motifs based on PSQQ or SPQQ. Other seemingly important variations include PSDQ, PSEQ and PSSQ (Marsh, 1992, Gastroenterology 1992:102:330-354).

CD is an immunological disease and shows a well defined HLA-association. A large majority of patients expresses the HLA-DQ2[α1*0501,β1*02] and/or HLA-DQ8[α1*0301,β1*302] molecules, and gluten specific HLA-DQ-restricted T-cells have been isolated from small intestinal biopsies of coeliac disease patients (Lundin et al, 1993, J. Exp. Med. 178:187-196). It is thought that this T cell reactivity against gluten peptides leads to disease. Moreover, that continuous exposure can lead to malignant transformation, resulting in cancer. The nature and sequence of the peptides that are recognised (and thus lay at the heart of the problem) by intestinally derived gluten-specific T-cells, however, is unknown.

The invention now provides a method to find and characterise peptides that are recognised by intestinally derived gluten-specific T-cells. The method comprises the selection of gluten-specific T-cells, stimulation of these cells with peptides (such as enzymatic digests or synthetic peptides) derived from gluten and selecting (assay fractions containing) bioactive peptides capable of stimulating said T-cells. In the experimental part, an example of the invention is provided wherein the method further comprises using (assay fractions of) peptides derived via pepsin/trypsin digestion of gluten which are purified by HPLC chromatograpy, and wherein a (fraction containing a) bioactive peptide is characterised and sequenced by mass spectrometric analyses. The invention provides a method to find and characterise a peptide that is recognised by an intestinally derived gluten-sensitive T-cell comprising establishing at least one gluten-sensitive T-cell clone and contacting clonal T-cells with a mixture of peptides derived from gluten characterised in that said mixture is fractionated whereby bioactive fractions that stimulate said clonal cells are selected and said bioactive fractions are (preferably repeatedly) used to contact said cells. Furthermore, a method is provided wherein bioactive fractions are subjected to mass spectrometric analysis and/or are selected by high pressure liquid chromotography. The invention also provides a peptide, and derivative thereof, that is specifically recognised by intestinally derived gluten-specific T-cells. Such a peptide is obtainable by a method provided by the invention and can be obtained from a wide range of prolamines, such as gliadines, secalins, hordeins and avenins. In the experimental part, an example of such a peptide provided by the invention is presented comprising a 35-mer gliadin fragment peptide 198-232, more preferably comprising the 25-mer gliadin peptide 198-222, preferably at least comprising the bioactive core of the 11-mer gliadin peptide 206-216 (SGQGSFQPSQQ). Another example of such a peptide provided by the invention is a 15-mer glutenin peptide GQQGYYPTSPQQSQQ, comprising a repetitive sequence found in the glutenine molecule. Surprisingly, it has now been found that such a glutenin-derived peptide is also responsible for CD. The invention also provides natural variants or functional derivatives having similar properties as a peptide provided by the invention, similar meaning in this application that they can stimulate gluten-sensitive T-cells, although the amount and extend of stimulation may differ. Such derivatives (which may be synthetic peptides but can also be derived of related prolamines) can be now obtained by various methods known in the art. The peptide and/or its derivatives can be incorporated in vaccines, in pharmaceutical formulations and they can be used in diagnostic test kits.

The invention also provides peptides and peptide preparations to be used to induce tolerance against celiac disease. For tolerance induction doses of the peptides provided by the invention can be given repeatedly, for instance intravenously, but other routes of administration may very well be suitable too. Another possibility is the repeated oral administration of the peptides. The peptides may be given alone, or in combination with other peptides, or as part of larger molecules, or coupled to carrier materials in any suitable excipients. On the basis of the peptide described herein immunological tools such as anti-idiotypic B-cells and/or T-cells and their receptors and antibodies can be produced by several methods based on *in vivo* or *in vitro* immunization protocols. A preferred embodiment of such immunological tools are those recognising a continous (linear) epitope formed by a peptide provided by the invention. Such epitopes can for example be provided by synthetic peptides. Such immunological tools can for example be used in diagnostic assays such as western blotting, ELISA test and ELISA spottests. Also, synthetic antibodies specifically reactive with a peptide provided by the invention, as for example obtainable by phage-display techniques, are now provided.

The invention also provides an analog (synthetic or natural variant) of the gluten derived peptide that is specifically recognised by intestinally derived gluten-specific T-cells which analog is an antagonist for the activity of T cells recognizing said peptide. Such an analog is especially well suited to be used in therapy against gluten sensitivity.

Furthermore the peptides according to the invention are used to prepare therapeutic agents capable of eliminating a subset of cells, directly or indirectly, especially gluten-sensitive T-cells. This means that an agent, which typically comprises a peptide according to the invention as recognised selectively by T-cells, which agent induces elimination of the cells recognising said peptide, is administered to the patient. Such an agent most typically comprises a toxic moiety.

Diagnostic applications are clearly within the skill of the art. They include, but are not limited to detection of gluten-sensitive T-cells by for example histology. Specific gene sequences (such as TCR sequences) can be detected with various methods known in the art, such as hybridization or amplification with PCR and the like. Immunological detection of peptides has also widely been practiced.

The invention also provides methods for the selection and/or production of cereals which bear less or no risk when included in the food of gluten-sensitive individuals. Such cereals, such as wheat, rye, barley, oats, and related grasses can now be selected for the presence or absence of prolamins comprising a peptide sequence that is specifically recognised by intestinally derived gluten-specific or sensitive T-cells. Such selected cereals can than be produced via agricultural and/or industrial methods into foodstuff for gluten-sensitive individuals.

Selection of such cereals can now be done by diagnostic techniques, including various methods known in the art, such as nucleic acid detection by hybridization or amplification with PCR and the like. Immunological detection by specific antibodies of such peptide sequences is now also possible. The invention thus also provides a diagnostic assay incorporating means and methods to detect the nucleic acid sequences or (poly)peptide sequences present or absent in cereals that bear relevance to gluten-sensitivity.

With such assays provided by the invention one can detect and select or produce cereals that are less of a risk or that are no risk to gluten-sensitive individuals because they comprise prolamins specifically lacking a peptide sequence, such as SGQGSFQPSQQ or GQQGYYPTSPQQSGQ or derivatives thereof, that is specifically recognised by intestinally derived gluten-specific or sensitive T-cells. By detecting cereals substantially lacking a peptide as provided by the invention, one can select and breed for new cereals that are fit for incorporation in the diet of a gluten-sensitive individual. Alternatively, one can now genetically modify the genome (of cells) of existing cereals to generate new cereals. Such modifications are generated by point-mutations in the nucleic acid sequence of the prolamine, encoding a peptide sequence, such as SGQGSFQPSQQ or GQQGYYPTSPQQSGQ or derivatives thereof, provided by the invention, or are generated by replacing such a sequence by recombinant techniques by another sequence not encoding a peptide sequence provided by the invention. Other recombinant techniques encompass the use of anti-sense nucleic acids.

With such an assay provided by the invention one can also detect and select cereals and prolamines for the presence of peptide sequences that are less of a risk or that are no risk to gluten-sensitive individuals, because they substantially lack a peptide that stimulates gluten-sensitive T-cells. A preferred embodiment provided by the invention is the immunological detection, preferably employing antibodies specifically recognising a (continous) peptide provided by the invention which allows easy detection of foodstuff containing prolamins that do not or only little contribute to gluten sensitivity related disease. In this way the invention provides cereals, comprising modified prolamines, that form the basis of a specialty food or specific diet for gluten-sensitive individuals. The invention is further explained in the experimental part below which cannot be seen as limiting the invention.

### Experimental part

### INTRODUCTION

Ingestion of gluten, a common dietary protein present in wheat, was already recognized as the cause of coeliac disease (CD) in 1953 (1). Gluten is a complex mixture of glutamine-and proline- rich glutenin and gliadin molecules, the latter of which is held responsible for disease-induction (2,3). The isolation of HLA-DQ restricted gluten-specific T-cells from the small intestine of a CD patient was first shown by the group of Lundin et al. in 1993 (4). The identification of the T- cell stimulatory epitopes, however, has been hampered by the vast heterogeneity and peculiar nature of the gluten proteins. By combining HPLC-purification steps with a T-cell bioassay and mass spectrometric analyses, we have now been able to identify the first gliadin epitope that is recognised by intestinally-derived T-cells from a CD patient.

### MATERIALS AND METHODS

### Antigens and peptides

A pepsin/trypsin digest of gluten was prepared essentially as described previously (4). Briefly, 1 g of gluten (FLUKA) was solubilized in 10 ml of 1M acetic acid and boiled for 10 minutes. Ten mg of pepsin (SIGMA) was first added and the mixture was incubated for 4 hours at 37°C. After pH adjustment to 7.8 and addition of 10 mg of trypsin (SIGMA), the mixture was incubated for another 4 hours at 25°C. Finally, 10 mg of trypsin inhibitor (SIGMA) was used to stop further trypsin activity, followed by dialysis of the mixture against a large volume of water. Protein concentration was determined using a BCA protein assay (PIERCE). Synthetic peptides were made as described previously (5).

### Generation of gluten-specific T cells

Exogenous antigens like gluten must be taken up by antigen presenting cells for degradation in intracellular compartments. This degradation results in the formation of peptides that bind to intracellular HLA-class II molecules. The class II-peptide complexes are subsequently transported to the cell surface for recognition by CD4 positive, HLA-class II-peptide specific T cells. The efficiency of the uptake of exogenous antigens, therefore, is a critical step towards the formation of antigenic class II-peptide complexes. In order to optimize the uptake of gluten we used a source of antigen presenting cells that are known to be extremely efficient in the endocytosis of exogenous antigens: GM-CSF/ IL-4 cultured human monocytes. These cells were cultured with the gluten preparation in order to allow for maximal formation of HLA class II-gluten peptide complexes and subsequently used for stimulation of T cells from small intestinal biopsies.

A small intentinal biopsy was taken from patient S, an adult CD patient on a gluten-free diet for several years. The patient gave informed consent to the study, which was approved by the hospital ethics committee. The patient was typed serologically to be HLA-DR3/4, DQ2/8, thus carrying both CD-associated DQ dimers. After treatment of the biopsy with 1 mM DDT (10 minutes) and 0.75 mM EDTA (4 hours) in calcium/magnesium-free HBSS (GIBCO) at 37°C under mild agitation, the biopsy was put into culture with autologous IL4/GM-CSF-cultured monocytes as a source of highly endocytic antigen presenting cells (APCs) (6). The APCs have been cultured in the presence of 250 µg/ml of the enzyme-treated gluten preparation of 48 hours before the biopsy was taken. After 5 and 10 days of coculture of the T cells and APCs, 20 U/ml r-IL2 (EUROCETUS, Amsterdam, The Netherlands) was added to the culture medium (RPMI 1640 [GIBCO] with 10% pooled inactivated human serum and 10 µg/ml gentamycin [GIBCO]. Subsequently, the cells were restimulated on a 8-days cycle with 20 U/ml r-IL2, 1 µg/ml PHA (MUREX BIOTECH, Dartford, UK), antigen and autologous monocyte-enriched irradiated PBMCs as APCs. T cell clones were established by limiting dilution (0.3 cell/well) in 150 µl culture medium in 96-well round-bottomed plates (FALCON), using 10⁵ allogeneic PBMC (3000 Rad), 1 µg/ml PHA, and 20 U/ml rIL-2. This procedure resulted in the establishment of several HLA-DQ-restricted T cell clones with a CD3+, CD4+, *TCRa*/*β*+phenotype. Proliferation assays were performed in duplicate or triplicate in 150 µl culture medium in 96-well flat-bottomed plates (FALCON) using 10⁴ T cells stimulated with 10⁵ PBMC (3000 Rad) in the absence or presence of antigen at several concentrations. After 48 hours, cultures were pulsed with 0.5 µCi of ³H-thymidine and harvested 18 hours thereafter.

### HPLC purification of the pepsin/trypsin digest of gluten

The enzymatic digest of gluten was purified in three successive rounds of HPLC chromatography as follows, ± 3 mg of gluten was purified on an analytical HPLC system (JASCO), using a gradient of 2% B/min (column Vydac 218TP1010, buffer B: acetonitrile, containing 0.1% trifluoric acid). Subsequently, bioactive fractions were further separated by micro-rpHPLC (SMART, Pharmacia), using a gradient of 0.25% B/min (column C2/C18 sc 2.1/10 [Pharmacia], buffer B: acetronile, containing 0.1% trifluoric acid). Finally, bioactive fractions were subfractionated by micro-rpHPLC, using a gradient of 0.25% B/min (buffer B: acetonitrile, 0.1% heptafluorobutyric acid).

### Mass spectrometry

Several attempts to sequence the peptides by the conventional Edman degradation method failed due to the presence of other, not biologically active, peptides in these fractions and the fact that the relevant peptides are glutamine rich. This severely complicated N-terminal sequencing by Edman degradation due to the formation of an N-terminal pyroglutamic acid residue, resulting in a non-sequencible N-terminus. Both these problems were circumvented by the use of tandem mass spectrometry since this technique is capable of sequencing a single peptide in a mixture of peptides and is not dependent on a free N-terminus.

HPLC fractions were monitored for their peptide content by matrix-assisted laser-desorption ionization time of flight mass spectrometry (MALDI-TOF-MS) on a Lasermat (Finnigan MAT, UK) as described previously (5). Electrospray ionization mass spectrometry was performed on a hybrid quadrupole-time of flight mass spectrometer, the Q-TOF (Micromass, Manchester, UK). The final bioactive fraction was introduced either by off-line nanoelectrospray, using type A needles (Micromass) or by flow injection analysis using the on-line nanoflow electrospray interface with an approximate flow rate of 200 nl/min. In the latter case 0.5-1.0 µl injections were done with a dedicated micro/nano HPLC autosampler, the FAMOS (LC Packings, Amsterdam, the Netherlands). MS/MS analyses were performed on the most abudant peptide peaks present in the bioactive fraction. Precursors (the triple and quadruple protonated molecules in case of the 35-mer) were selected with the quadrupole and fragments were collected with high efficiency with the orthogonal time of flight mass spectrometer. The collision gas applied was argon (pressure 4x10⁻⁵ mbar) and the collision voltage approximately 30 V.

### Database searching

The program PeptideSearch was used for sequence elucidation. This program has been developed to identify sequences in databases using mass spectral information. This program is available upon request from the authors of the program (7).

### RESULTS

### Isolation of HLA-DQ-restricted gluten-specific T cells from the small intestinal mucosa of a CD patient

Gluten-specific T cells were isolated from a small intestinal biopsy of patient S using autologous IL4/GM-CSF-cultured monocytes as a source of highly endocytic APCs (see Materials and Methods section). Several gluten-specific T cell clones (TCCs) were established by limiting dilution. The gluten-specific proliferative capacity of all gluten-specific TCCs could only be blocked by the HLA-DQ spefific moAB SPV-L3 (8) and not by the HLA-DR specific moAB B8.11.2 (two representative TCCs are shown in Table 1). Next, the reactivity of the TCCs was investigated using a panel of HLA-typed PBMCs in the presence of the gluten digest. Based on these results we could divide our TCCs into two categories: the first type of TCCs recognized gluten in the context of HLA-DQ2 (TCC 11 in Table 1), whereas the second type of TCCs recognized gluten via HLA-DQ8 (TCC 2 in Table 1). In line with this, the HLA-DQ3 (DQ7/8/9) specific moAb IVD12 (9) only blocked the proliferation of the DQ8-restricted type of TCCs (not shown).

### Identification of an HLA-DO8-restricted gliadin-derived T cell epitope

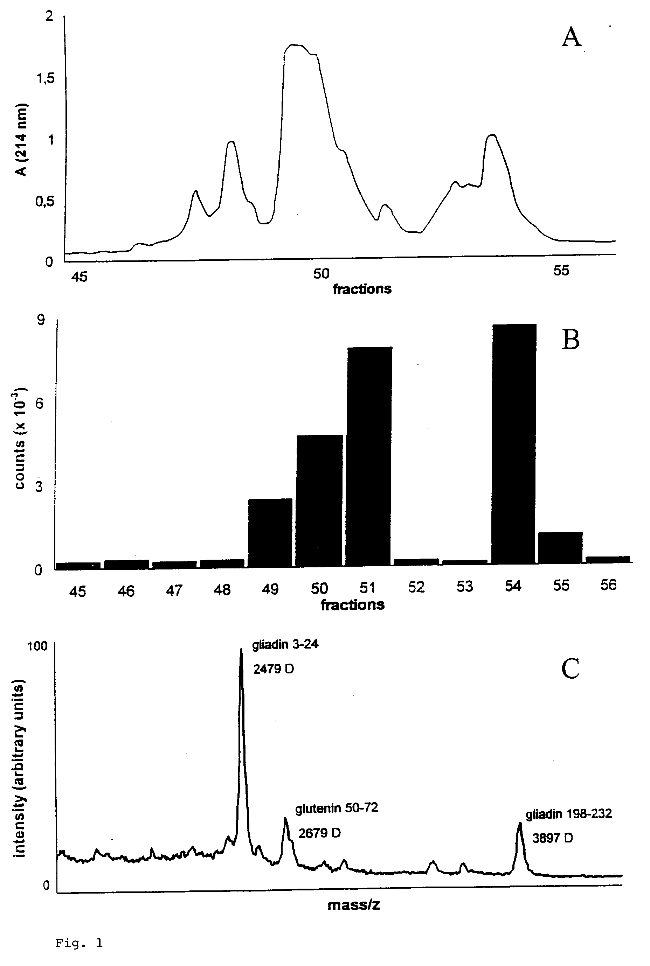

To identify the gluten-derived epitopes that are recognized by the TCCs, the pepsin/trypsin digest of gluten was purified in successive rounds of HPLC chromatography (see Materials and Methods section). After each purification step the peptide content of the fractions was monitored by MALDI-TOF mass spectrometry and the fractions were screened for their bioactivity in a standard proliferation assay. After three rounds of HPLC purification (Fig.1A), one of the fractions (# 54) that strongly stimulated one of the TCCs (TCC 2, DQ8-restricted) (Fig. 1B) was found to contain three dominant peaks with masses of 2479, 2769, and 3897 Da (Fig. 1C).

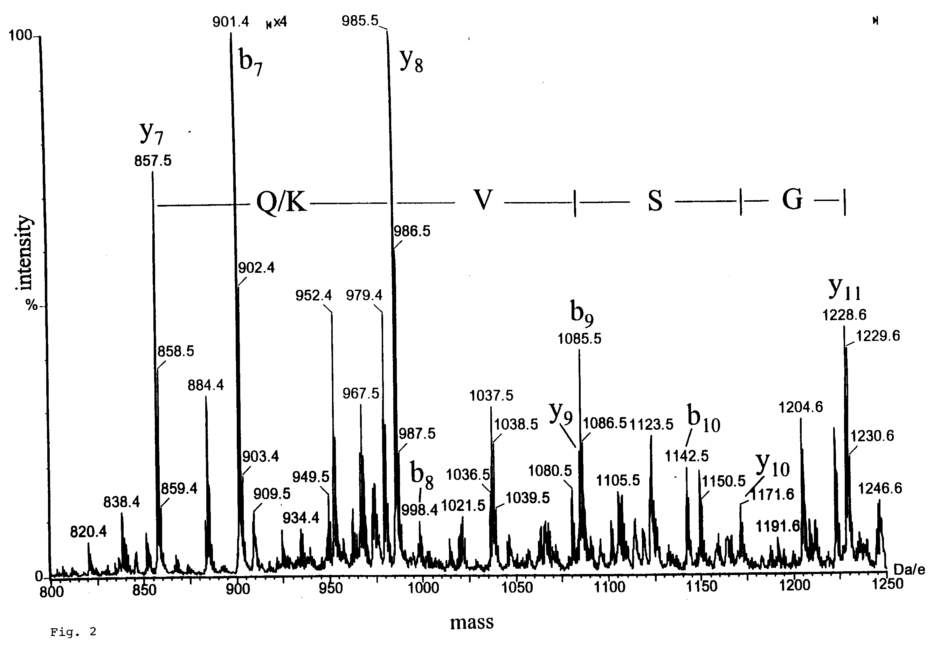

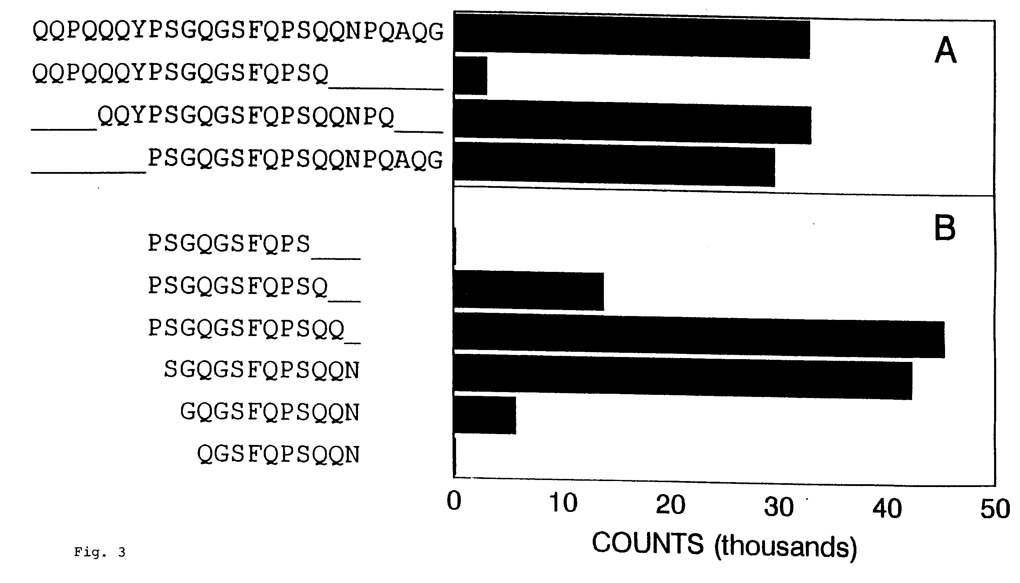

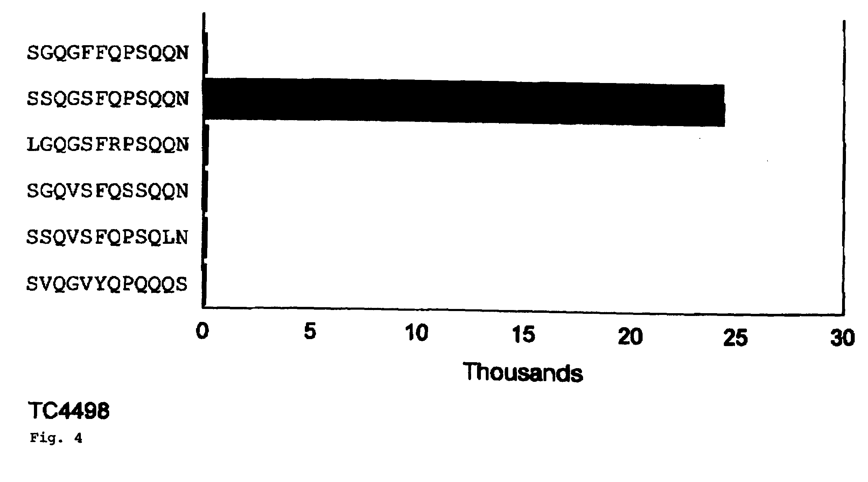

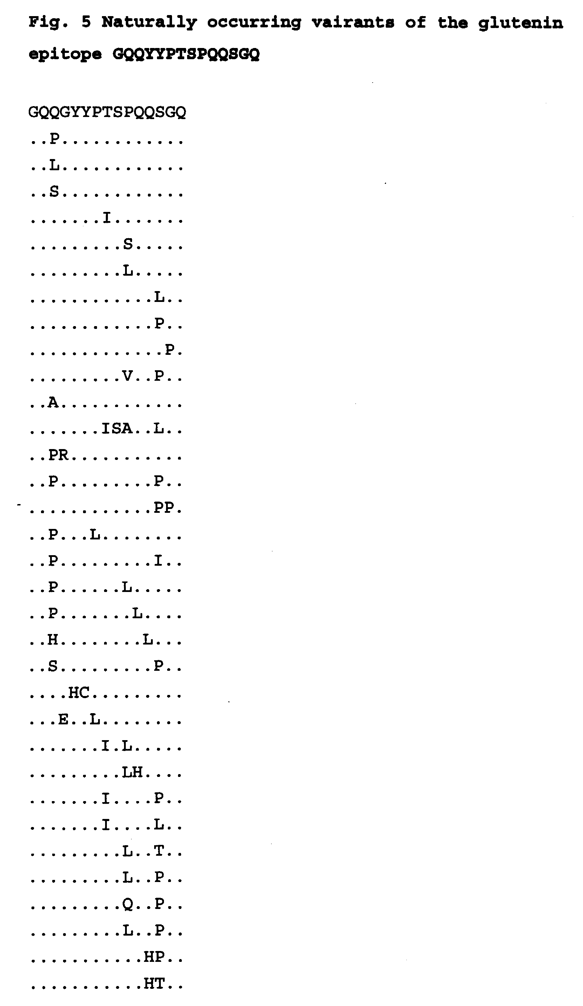

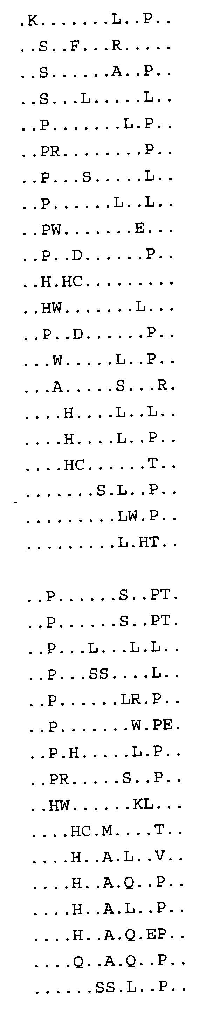

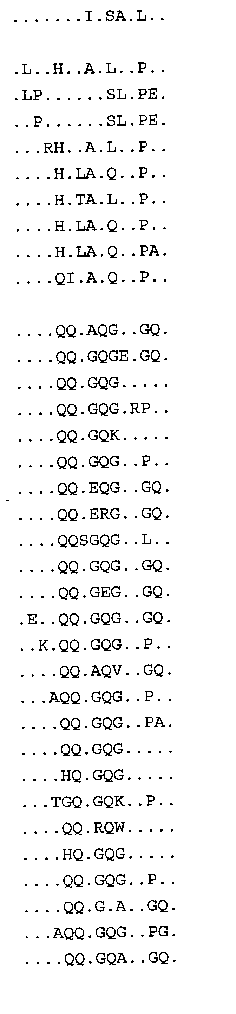

Subsequently, these peptides were individually subjected to MS/MS analysis in order to obtain amino acid sequence information, using a highly sensitive hybrid Quadrupole-TOF mass spectrometer. For each peptide partial sequence information was obtained, upon which database-searching resulted in the unambiguous identification of the amino acid sequences of all three peptides. One of the peptide peaks (2679 Da) was found to represent a glutenin sequence (residues 50-72), whereas the other two peaks (2479 and 3897 Da) were derived from gliadin (residues 3-24 and residues 198-232 respectively) (Fig. 1C). Part of the MS/MS fragmentation pattern of the 3897 Da fragment together with the database search-strategy is shown in Fig. 2. For elucidation of the active 35-mer gliadin peptide 198-232 the search string (857.5) Q/K,V,S (1171.6) was used (Fig. 2). Only one hit in the non-redundant protein database (8 april 1997, 234,000 entries) was found corresponding to gliadin(-like) proteins: gliadin gda09 (SwissProt Acc. Number P18573). Close inspection of the theoretical MS/MS spectrum and the measured MS/MS spectrum revealed that these matched (not shown). Using synthetic versions of the three identified peptides we found that only one of the peptides (corresponding to the 3897 Da peak) stimulated TCC 2 (results not shown). The stimulating capacity was found in a peptide corresponding to the first 25 amino acids of this gliadin fragment (residues 198-222) (Fig. 3A). Next, we determined the core peptide that is required to induce optimal proliferation. For this purpose overlapping 18-mer peptides, covering the stimulating 25-mer fragment, were tested and the results showed that the epitope is located in the central region of the gliadin 198-222 fragment (Fig 3A). Further truncation analysis demonstrated that the deletion of residues S₂₀₆ and Q₂₁₆ dramatically and/or reduced the stimulating capacity of the gliadin variants, demonstrating that the epitope with full biological activity at least comprises residues 206-216 (SGQGSFQPSQQ) (Fig. 3B) and residual activity resides in 207-215. Six natural variants of this peptide, found in related gliadin sequences, were also tested with TCC2, five of which were unreactive (Fig. 4). Further analysis of the HPLC fractions resulted in the identification of amino acid sequences of several additional gliadin and glutenin derived peptides. Synthetic versions of all these peptides were tested for their capacity to stimulate the gluten specific T cell clones. This led, among others, to the identification of a peptide (GQQGYYPTSPQQSGQ) that stimulated TCC12, an HLA-DQ8 restricted T cell clone distinct from TCC2. The peptide that stimulates TCC12 represents a repetative sequence found in the glutenin. Moreover, multiple variants of this sequence are found in the glutenins, see Fig. 5.

### DISCUSSION

We fractionated a crude gluten preparation by rpHPLC fractionation and screened the fractions for bioactivity in a T cell proliferation assay using gluten specific, HLA-DQ restricted T cell clones and appropriate HLA-DQ positive antigen presenting cells. Repeated HPLC purifications using two distinct eluents, one containing trifluoroacetic acid and the other heptafluorobutyric acid, were necessary to obtain fractions that contained only few peptide species with a distincts mass, thus allowing mass spectrometric analysis of these individual peptides. Mass spectrometry was subsequently used to obtain amino acid sequence information on the peptides present in the T cell stimulatory HPLC fractions. Mass spectrometry has been successfully applied to the identification of T cell stimulatory, HLA-class I restricted peptides. HLA-class I bound peptides are usually 8 to 10 amino acids long and therefore their amino acid sequence can be relatively easily determined by tandem mass spectrometry. In contrast, the peptides described herein are, in general, much longer, between 25 and 35 amino acids. This severely complicates the identification of their amino acid sequence by tandem mass spectrometry. It was therefore essential to combine the results of the mass spectrometric analysis with data base searches in order to obtain the complete amino acid sequences of the T cell stimulatory peptides. Their identity was confirmed by comparing the original fragmentation patterns generated with the tandem mass spectrometer with those generated using synthetic versions of the identified peptides. These patterns were found to be identical.

The large majority of human CD4 positive T cells recognize antigenic peptides when these are bound to HLA-DR molecules, i.e. these T cells are HLA-DR restricted. Only few CD4 positive T cells recognize antigenic peptides when these are bound to the related HLA-DQ molecules, i.e. are HLA-DQ restricted. We present the first example of the characteriztion of an antigenic, HLA-DQ restricted T cell epitope with the aid of HPLC purification and subsequent mass spectromectric analysis.

We describe the first identification of a gliadin-derived peptide that is recognized by a T-cell clone isolated from the small intestinal mucosa of a CD patient. The epitope (gliadin residues 198-232) most likely results from pepsin digestion of the gliadin molecule, which suggests that the fragment can be generated in the stomach and be recognised by T cells in the small intestine.

Furthermore, we describe a second epitope, which is the first identification of a glutenin-derived peptide that is recognised by a T-cell clone isolated from a CD patient. This indicates that contrary to what has generally been thought before, the disease inducing activity not only resides in the gliadins but that glutenin specific T cells play a role as well. Lundin et al. (10) reported that the combination of heating at low pH and pepsin treatment increased the stimulatory capacity of a crude gliadin preparation for an HLA-DQ2 restricted intestinal T cell clone. The authors suggested that this enhanced stimulatory capacity could be explained by the effect of pepsin treatment on the solubility of gluten and the putative deamidation of glutamine residues (the conversion of glutamine to glutamic acid) as a result of heating at low pH (11). However, our data indicate that pepsin treatment is necessary for the generation of certain gliadin-derived T cell epitopes. In adition, no evidence was found for deamidation of glutamine residues in any of the identified peptides in the present study. We did observe a putative modification in certain MS/MS fragmentation patterns, leading to a loss of 17 Da in a region of multiple glutamine residues (not shown). Although the nature of this putative modification is still unclear, it may very well represent a ring formation between the side chains of two adjacent glutamine residues, resulting in the loss of NH₃ (corresponding to 17 Da). Whereas in the case of the present epitope amino acid modifications have not yet been demonstrated to play a role in TCR recognition, it is conceivable that modified residues in gliadins are essential parts of epitopes recognized by gluten-specific TCCs.

In previous studies two gliadin-derived peptides have been identified that are recognized by T cell clones isolated from the periheral blood of patients (12,13). There is no indication, however, that these peptides are also stimulatory for T cells in the gut. Interestingly, these peptides are located in the N-terminal region of gliadin whereas our current T cell epitope (gliadin residues 198-232) is located in the C-terminal region of gliadin and represents a unique sequence that is found in only one of the many gliadin variants (gda09), and another of our T-cell epitopes is not derived of gliadin at all, but of glutenin instead. The minimal peptide necessary to stimulate the T cell clone TCC2 is 9-11 amino acids long and this sequence is found in only two gliadin variants (gda04 and gda09). The length of this minimal peptide is in accordance with the length of most minimal antigenic determinants recognized by class II restricted T cells (14). This core peptide contains the amino acid sequence PSQQ at its C-terminus, representing one of the repetitive motifs within gliadin that has been suggested to be responsible for toxicity in CD patients (2), however, this sequence, and related sequences, also occur frequently in peptides that were shown not to be specific for gluten-sensitive T-cells (4).

On the basis of the identified peptide sequences peptides with antagonistic properties that can abrogate the response to the stimulatory gluten peptides are designed. For this purpose substitution analogs of the stimulatory peptides are synthesized that can be tested for their capacity to stimulate the gluten specific T cell clones. Non-stimulatory analogs can be tested for their capacity to block the response to the agonist. Gluten consists mainly of gliadins and glutenins. Multiple variants of both are known, many of which are present in any given wheat strain. As a result of this, several natural variants of the identified DQ8 restricted T cell epitopes exist. In the case of the gliadin derived epitope this is limited to 6, but at least 80 variants of the glutenin epitope have been identified. We can now determine which of these variants are recognized and which of these naturally occurring variants influence the response to the stimulatory peptide (the agonist).

We only observed DQ2- and DQ8-restricted, gluten-specific responses in the T cell clones derived from the small intestine. These findings further strengthen the hypothesis that the mechanism behind the DQ2/DQ8 association in CD is the presentation of gluten-derived peptides to T cells in the small intestine (19). Lundin et al. (10) reported that a set of HLA-DQ restricted, gluten-specific TCCs isolated from the small intestinal mucosa of five CD patients displayed a heterogeneous pattern of reactivity, suggesting that stimulation of different gliadin-specific T cells in the intestine may be important in the pathogenesis of CD. We also observed that the DQ2-restricted T cell clones recognized different HPLC fractions of the pepsin/trypsin digest than the DQ8-restricted T cells. Additional gluten derived T-cell epitopes thus exist, the identification of which is now also possible by the method provided by the invention.

It is conceivable that the dominant production of IFN_{y} by gluten-reactive T cells (20,21) in the small intestinal mucosa can be involved in the development of the coeliac lesion. Knowledge of the disease-inducing epitopes of gliadin or glutenin is a key step towards the development of strategies to prevent T cell recognition of these peptides, thereby interfering with the production of cytokines and subsequent tissue destruction. Furthermore, this knowledge raises the possibility of future manipulation of for example the wheat genome in order to produce wheat products that are tolerated by gluten-sensitive individuals.

### REFERENCES

1. Van de Kamer, J.H., H.A. Weijers, and W.K. Dicke, 1953. Coeliac Disease IV. An investigation into the injurious constituents of wheat in connection with their action on patients with coeliac disease. *Acta. Paediatr*. 42:22.-231.
2. Marsh, M.N. 1992. Gluten, major histocompatibility complex, and the small intestine. *Gastroenterology* 102:330-354.
3. Trier, J.S. 1991. Celiac sprue. *N. Engl.J.Med*. 325:1709-1719.
4. Lundin, K.E.A., H. Scott, T. Hansen, G. Paulsen, T.S. Halstensen, O. Fausa, E. Thorsby, and L.M. Sollid. 1993. Gliadin-specific, HLA-DQ2 restricted T cells isolated from the small intestinal mucosa of celiac disease patients. *J*. *Exp. Med*. 178:187-196.
5. Van de Wal, Y., Y.M.C. Kooy, J.W. Drijfhout, R. Amons, and F. Koning. 1996. Peptide binding characteristics of the coeliac disease-associated DQ(*a*1*0501,*β*1*0201) molecule. *Immunogenetics* 44:246-253.
6. Sallusto, F and A. Lanzavecchia. 1994. Efficient presentation of soluble antigen by cultured human dendritic cells is maintained by granulocyte/macrophage colony-stimulating factor plus interleukin-4 and downregulated by tumor necrosis factor-*a*. *J.Exp. Med.* 179:1109-1118.
7. Mann, M. and M. Wilm. 1994. Error-tolerant identification of peptides in sequence databases by peptide sequence tags. *Anal. Chem.* 66:4390-4399.
8. Spits, H., J. Borst, M.J. Giphart, J. Colligan, C. Terhorst, and J. De Vries. 1984. HLA-DQ antigens can serve as recognition elements for human cytotoxic T lymphocytes. *Eur*. *J. Immunol* 14:299-304.
9. Giles, R.C., G. Nunez, C.K. Hurley, A. Nunez-Roldan, R. Winchester, P. Stastny, and J.D. Capra. 1983. Structural analysis of a human I-A homologue using a monoclonal antibody that recognizes an MB3-like specificity. *J. Exp. Med.* 157:1461-1470.
10. Lundin, K.E.A., L.M. Sollid, D. Anthonson, O. Norén. Ø. Molberg, E. Thorsby, and H. Sjöström. 1997. Heterogeneous reactivity patterns of HLA-DQ-restricted, small intestinal T cell clones from patients with celiac disease. *Gastroenterology* 112:752-759.
11. Bollecker, S. and Y. Popineau. 1991. Functional properties of deamidated gliadins, 4^{th} International Workshop on Gluten Proteins, Winnipeg, Manitabo, Canada: 29-41.
12. Gjertsen, H.A., K.E.A. Lundin, L.M. Sollid, J.A. Eriksen and E. Thorsby. 1994. T cells recognize a peptide derived from *a*-gliadin presented by the celiac disease associated HLA-DQ2 heterodimer. *Hum. Immunol*. 39:243-252.
13. Franco, A., E. Appella, M.F. Kagnoff, Y. Chowers, K. Sakaguchi, H.M. Grey, and A. Sette. 1994. T cell response to A-gliadin in celiac disease. Differential processing and presentation capacities of Epstein-Barr transformed B cells and fibroblasts. *Clin*. *Immunol. Immunopathol*. 71_75-81.
14. Panina-Bordignon, P., A. Tan, A. Termijtelen, S. Demotz, H|G. Corradin, and A. Lanzavecchia: 1989. Universally immunogenic T cell epitopes: promiscuous binding to human MHC class II and promiscuous recognition by T cells. *Eur. J*. *Immunol*. 19:2237-2242.
15. de Ritis, G., S. Auricchio, H.W. Jones, E.J. Lew, J.F. Bernardin, and D.D. Kasarda. 1988. In vitro (organ culture) studies of the toxicity of specific *a*-gliadin peptides in celiac disease. *Gastroenterology* 94:41-49.
16. Wieser, H., H.D. Beleitz, D. Idar and A. Ashkenzai. 1986. Coeliac activity of gliadin peptides CT-1 and CT-2. *Lebensm*. *Unters Forsch* 182:115-117.
17. Kagnoff, M.F., R.K. Austin, J.J. Hubert, J.E. Bernardin, and D.D. Kasarda. 1984. Possible role for a human adenovirus in the pathogenesis of celiac disease. *J. Exp. Med.* 160:1544-1557.
18. Karagiannis, J.A., J.D. Priddle, and D.P. Jewell. 1987. Cell-mediated immunity to a synthetic gliadin peptide resembling a sequence from adenovirus 12. *Lancet* 884-886.
19. Lundin, K.E.A., H.A. Gjertsen, J. Scott, L.M. Sollid, and E. Thorsby. 1994. Function of DQ2 and DQ8 as HLA susceptibility molecules in celiac disease. *Hum. Immunol.* 41:24-27.
20. Nilsen, E.M., K.E. Lundin, P. Krajci, H. Scott, L.M. Sollid and P. Brandtzaeg. 1995. Gluten-specific, HLA-DQ restricted T cells from coeliac mucosa produce cytokines with TH1 or TH0 profile dominated by interferon gamma. *Gut* 37:766-776.
21. Nilsen, E.M., H.A. Gjertsen, K. Jensen, P. Brandtzaeg, and K.E. Lundin. 1996. Gluten activation of peripheral blood T cells induces a TH0-like cytikine pattern in both coeliac patients and controls. *Clin. Exp.Immunol*. 41:295-303.

**Table 1**

| TCC 11 is restricted via HLA-DQ2; TCC2 is restricted via HLA-DQ8 | | | | | | | |
|---|---|---|---|---|---|---|---|
| | | TCC 2 | | | TCC11 | | |
| donor | HLA-DQ type | gluten digest | gluten digest +B8.11.2 | gluten digest +SPV-L3 | gluten digest | gluten digest +B8.11.2 | gluten digest +SPV-L3 |
| FK | 2/8 | **10.7** | **8.5** | 0.1 | **8.1** | **7.9** | 1.5 |
| YK | 1/8 | **13.1** | **10.9** | 0.1 | 0.4 | 0.4 | 0.1 |
| EB | 6/8 | **7.5** | **11.9** | 0.1 | 0.2 | 0.1 | 0.1 |
| JP | 5/8 | **12.0** | **9.7** | 0.1 | 0.1 | 0.1 | 0.1 |
| ES | 6/8 | **9.8** | **8.4** | 0.2 | 0.2 | 0.1 | 0.2 |
| BSM | 8 | **28.1** | **19.7** | 1.0 | 0.5 | 0.1 | 0.1 |
| CA | 2 | 0.4 | 0.1 | 0.1 | **5.4** | **5.4** | 0.9 |
| DE | 2 | 0.1 | 0.1 | 0.1 | **7.3** | **7.5** | 0.7 |

T cell responses of two types of glucten-specific TCCs. 10⁴ T cells of the indicated TCCs were incubated with 10⁵ PBMCs (3000 Rad) from several donors in the presence of 0.7 mg/ml of a pepsin/trypsin digest of gluten. Blocking experiments were performed with the anti-DR moAb B8.11.2 and the anti-DQ-specific moAb SPV-L3. Values show mean cpm (x 10³) of duplicate cultures. Values considered positive are displayed in bold.

### FIGURE LEGENDS

Fig. 1: HPLC purification of the gluten-digest combined with a T cell bioassay and mass spectral analysis. [A] rpHPLC profile of the pepsin/trypsin digest of gluten after three rounds of purification: [B] stimulatory capacity of the collected rpHPLC fractions, shown in [A]. 10⁴T cells of TCC 2 were stimulated with 10⁵PBMC(3000 Rad from an HLA-DQ2/DQ8 positive donor in the presence of 2 µl of each rpHPLC fraction (total fraction size: 100 µl). Values show mean cpm (x10⁻³) of duplicate cultures. SD is <10%. [C] MALDI mass spectrometry profile (Lasermat) of the bioactive fraction 54 (see [A] and [B]).
Fig.2: Partial product ion mass spectrum of the 35-mer gliadin peptide (residues 198-232). The spectrum displays two series of peaks, the so-called b-ions and y-ions, that allow the sequence to be elucidated. A part of the y-ion series consists of the masses 857.5, 985.5, 1084.5 and 1171.6, which corresponds to the partial sequence Glutamine/Lysine (Q/K), Valine (V), Serine (S). This information, together with the total mass of the 35-mer peptide (3897 Da) was put in the program PeptideSearch. This led to several hits, but only one gliadine-related peptide was found, gda09 198-232.
Fig. 3: Truncation analysis of gliadin peptide 198-222. 10⁴ T cells (TCC 2) were stimulated with 10⁵ PBMC (3000 Rad) from an HLA-DQ2/DQ8 positive donor in the presence of 7.5 µg/ml of the indicated peptide sequences. Amino acids are shown as single-letter codes. Panel A shows the results of a set 18-mer peptides, covering the gliadin 198-222 sequence. Panel B shows the results of a set of truncated variants of the central portion of gliadin 198-222. Values show mean cpm (x 10⁻³) of truplicate cultures. SD is < 10%.
Fig. 4 Recognition of natural variants of T-cell epitope SGQGSFQPSQQ.
Fig. 5: Naturally occurring variants of the glutenin epitope GQQGYYPTSPQQSGQ.

## Claims

1. A method to find and characterise a peptide that is recognised by an intestinally derived gluten-sensitive T-cell comprising establishing at least one gluten-sensitive T-cell clone and contacting clonal T-cells with a mixture of peptides derived from gluten characterised in that said mixture is fractionated whereby bioactive fractions that stimulate said clonal cells are selected.

2. A method according to claim 1 further comprising repeated fractionating said bioactive fractions and selecting the resulting bioactive fractions.

3. A method according to claim 1 or 2 wherein said bioactive fractions are subjected to mass spectrometric analysis.

4. A method according to claim 1, 2, or 3 wherein said fractions are selected by high performance liquid chromotography.

5. A peptide obtainable by a method according to claim 1, 2, 3, or 4.

6. A peptide according to claim 5 which can be obtained from a prolamine, preferably a glutenin, a gliadin, secalin, hordein or avenin.

7. A peptide constituting a T-cell epitope obtainable from gliadin comprising the sequence SGQGSFQPSQQ or a derivative thereof having similar properties.

8. An immunogenic polypeptide obtainable from gliadin comprising the sequence SGQGSFQPSQQ or a derivative thereof having similar properties.

9. A peptide constituting a T-cell epitope obtainable from glutenin comprising the sequence GQQGYYPTSPQQSGQ or a derivative thereof having similar properties

10. An immunogenic polypeptide obtainable from glutenin comprising the sequence GQQGYYPTSPQQSGQ or a derivative thereof having similar properties.

11. Analog of a peptide according to any one of claim 5 to 10, which is an antagonist for the activity of T-cells recognising said peptide.

12. A pharmaceutical formulation comprising an epitope or a polypeptide according to any one of claims 5 to 10, preferably for inducing tolerance.

13. Peptide or polypeptide according to any one of claims 5 to 11 for use as a medicine.

14. Use of a peptide or polypeptide according to claims 5 to 11 in the preparation of a medicament for the induction of gluten tolerance or to treat gluten-sensitivity.

15. A method for the elimination of a group of gluten-sensitive T-cells presenting a peptide in the context of HLA-DQ according to any of one of claims 5 to 10, whereby elimination is induced directly or indirectly by specific recognition of said peptide in said context.

16. Method for the generation of antibodies, T cell receptors, anti-idiotypic B-cells or T-cells, comprising the step of immunisation of a mammal with a peptide or a polypeptide according to any one of claim 5 to 10.

17. Antibodies, T-cell receptors, B-cells or T-cells obtainable by the method of claim 16.

18. A diagnostic assay comprising means to detect a peptide sequence according to any of claims 5 to 11 or to detect a nucleic acid encoding a peptide according to any of claims 5 to 11.

19. A method to select a cereal substantially lacking at least one peptide sequence according to any of claims 5 to 10 which method comprises using an assay according to claim 18.

20. A method to select a cereal comprising at least one peptide sequence according to claim 11 which method comprises using an assay according to claim 18.

21. A cereal selected by a method according to claim 19 or 20.

22. A method to select a prolamine, preferably a gliadin, secalin, hordein or avenin, substantially lacking at least one peptide sequence according to any of claims 5 to 10 which method comprises using an assay according to claim 18.

23. A method to select a prolamine, preferably a gliadin, secalin, hordein or avenin, comprising at least one peptide sequence according to claim 11 which method comprises using an assay according to claim 18.

24. A prolamine selected by a method according to claim 22 or 23.

25. A cereal cell in which its nucleic acid has been genetically modified to encode a prolamine, preferably a gliadin, secalin, hordein or avenin, substantially lacking at least one peptide sequence according to any of claims 5 to 10

26. A cereal cell in which its nucleic acid has been genetically modified to encode a prolamine, preferably a gliadin, secalin, hordein or avenin, comprising at least one peptide sequence according to claim 11.

27. A prolamine derived from a cereal cell according to claim 25 or 26.

28. A cereal according to claim 21 and/or a prolamine according to claim 24 or 27 for inclusion in a diet for a gluten-sensitive individual.

29. A foodstuff for a gluten-sensitive individual comprising a cereal according to claim or prolamine according to claim 24 or 27.
